# EUROPÄISCHE PATENTSCHRIFT

(11) **EP 0 634 671 B1**
(45) Veröffentlichungstag und Bekanntmachung des Hinweises auf die Patenterteilung: **18.08.1999**
(21) Anmeldenummer: 94108335.4
(22) Anmeldetag: 30.05.1994
(51) Int. Cl.: G01T 1/164, A61B 6/03, A61B 6/14

(54) **Zeilendetektor-Kamera für die Verwendung bei insbesondere zahnärztlichen Röntgendiagnostikgeräten**
Line detector camera for application particularly in dental X-ray diagnostic devices
Caméra à détecteur des lignes utilisée notamment pour les appareils de radiodiagnostic dentaire

(30) Priorität: 06.07.1993 DE 4322484
(43) Veröffentlichungstag der Anmeldung: 18.01.1995
(73) Patentinhaber: Sirona Dental Systems GmbH, 64625 Bensheim (DE)
(72) Erfinder: Heubeck, Erich, D-64625 Bensheim (DE); Günther, Werner, D-64625 Bensheim (DE); Schulze-Ganzlin, Ulrich, Dipl.-Ing., D-64653 Lorsch (DE); Döbert, Michael, D-64653 Lorsch (DE); Plötz, Josef, Dr. Dipl.-Phys., D-64625 Bensheim (DE)
(74) Vertreter: Isenbruck, Günter, Dr.

(56) Entgegenhaltungen:
- EP-A- 0 166 567
- EP-A- 0 194 743
- EP-A- 0 279 293
- WO-A-81/00457

## Beschreibung

Bei Röntgendiagnostikgeräten ist es üblich, vor dem durchstrahlenden Körperteil (Zahn, Kiefer, Schädel, Mamma) eines Patienten eine dem Objekt angepaßte Kassette zur Aufnahme eines Röntgenfilmes anzuordnen. Will man beispielsweise vom Kiefer eine Panorama-(PAN-)Aufnahme erstellen, so ist eine andere Filmkassette mit einem anderen Filmformat erforderlich, als wenn beispielsweise vom Schädel eines Patienten eine Fernröntgen-(Ceph-)Aufnahme erstellt werden soll.

In der zahnärztlichen Röntgentechnik ist es bekannt, mit ein und demselben Grundgerät alternativ PAN- und Ceph-Aufnahmen machen zu können. Für Ceph-Aufnahmen wird an das Grundgerät ein Auslegerarm mit einem Schädelhalter und einer dem Objekt entsprechend angepaßten Filmkassette adaptiert. Wie bereits erwähnt, sind für beide Aufnahmearten unterschiedliche Filmkassetten und Filmformate erforderlich (EP-0 229 308 und EP-0 262 522).

Bekanntgeworden sind außerdem zahnärztliche Röngendiagnostikgeräte, bei denen anstelle eines Röntgenfilms ein strahlungsempfindlicher Sensor, z.B. ein CCD-Sensor, mit davor angeordneter Szintillationsschicht verwendet werden kann. Die bisher bekanntgewordenen Geräte dieser Art sind jedoch nur zur Erstellung von PAN-Aufnahmen geeignet (EP-0 279 294).

Mit der Erfindung wird eine Zeilendetektor-Kamera vorgestellt, die für Aufnahmen unterschiedlicher Körperteile einsetzbar ist, die im besonderen sowohl für PAN- als auch für Ceph-Aufnahmen verwendbar ist. Die Zeilendetektor-Kamera enthält elektrische und mechanische Anschlußmittel für den lösbaren Anschluß an einen entsprechenden Halter eines PAN- und/oder Ceph-Gerätes. Je nachdem, ob das Röntgendiagnostikgerät für die eine oder andere oder für beide Aufnahmearten vorgesehen ist, können mit nur einer Kamera die vorgenannten Aufnahmen erstellt werden. Im Falle einer PAN-Aufnahme kann die Kamera senkrecht am Halter, im Falle einer Ceph-Aufnahme kann sie senkrecht oder waagerecht am Halter befestigt werden. Im Gegensatz zu den bisher notwendigen, zwei unterschiedlich gestalteten Kassetten und Kassettenhaltern ist nurmehr eine einzige Kamera und jeweils baugleiche Halter vorzusehen.

Weitere vorteilhafte Ausgestaltungen und Weiterbildunqen der Erfindung ergeben sich aus den Unteransprüchen und der nachstehenden Beschreibung eines Ausführungsbeispieles.

### Es zeigen:

Figur 1 eine Prinzipdarstellung eines zahnärztlichen Röntgendiagnostikgerätes zur Erstellung von PAN-Aufnahmen,
Figur 2 das Gerät nach Figur 1, modifiziert zur Erstellung von Ceph-Aufnahmen,
Figur 3 die für Ceph-Aufnahmen vorgesehene Vorrichtung in schaubildlicher Darstellung,
Figur 4 die Vorrichtung nach Figur 3 in Frontansicht, teilweise im Schnitt,
Figur 5 eine Ausführungsform einer Zeilendetektor-Kamera mit zugehörigem Halter in schaubildlicher Explosionsdarstellung,
Figur 6 die Zeilendetektor-Kamera im Querschnitt, entlang der Linie VI-VI in Figur 5,
Figur 7 ein Blockschaltbild zur Ansteuerung der Verstellmotoren.

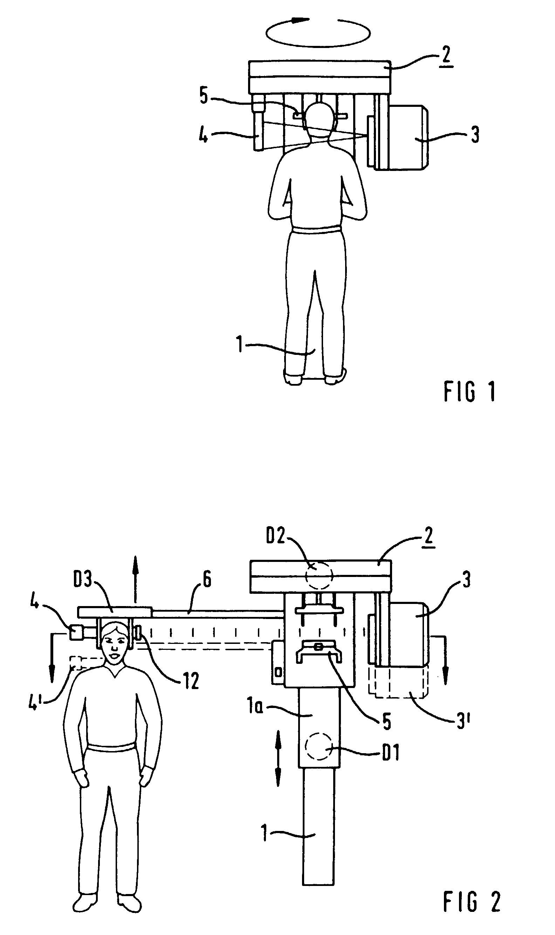

Die Figur 1 zeigt in einer Prinzipdarstellung ein zahnärztliches Röntgendiagnostikgerät zur Erstellung von Panorama-Schichtaufnahmen, nachfolgend kurz mit PAN-Aufnahmen bezeichnet. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgenzeilenkamera 4 ist. Mit 5 ist eine (erste) Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der EP-0 229 308 beschrieben.

Die Figur 2 zeigt das gleiche Grundgerät, bestehend aus höhenverstellbarer Tragsäule 1, Dreheinheit 2 und Röntgenstrahler 3, ergänzt jedoch durch eine am Gerät adaptierbare Vorrichtung, mit der sich Schädelfernaufnahmen, nachfolgend kurz Ceph-Aufnahmen, erstellen lassen. Bevor die genannte Vorrichtung näher erläutert wird, sei noch erwähnt, daß die Tragsäule 1 mittels eines Antriebes D1 in der angegebenen Pfeilrichtung höhenverstellbar ausgebildet ist, daß weiterhin in bekannter Weise die Dreheinheit 2 mittels eines oder mehrerer Antriebe D2 gedreht und geschwenkt werden kann, um eine PAN-Aufnahme machen zu können. Einzelheiten hierzu ergeben sich aus der bereits genannten EP-0 229 308.

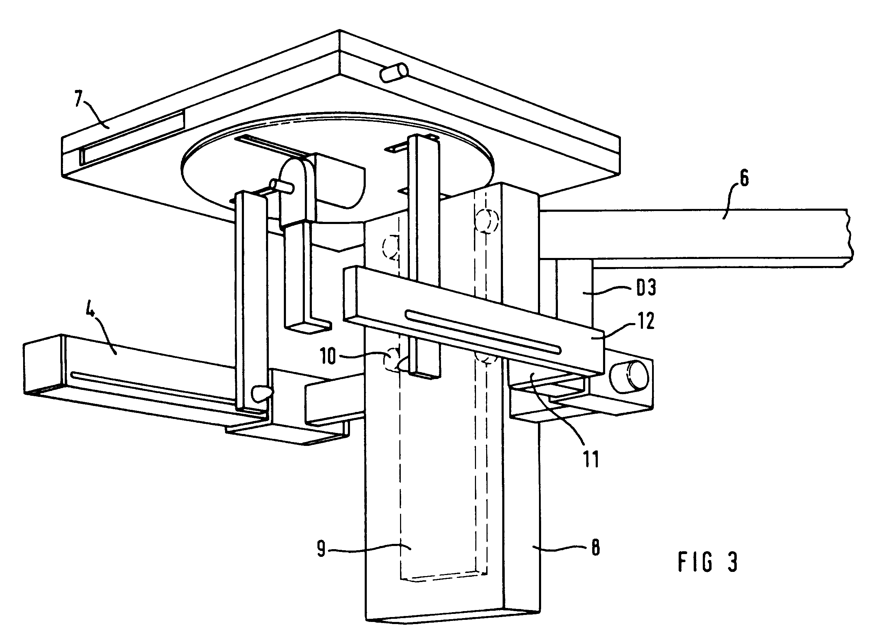

Die Figur 3 zeigt die vorgenannte Vorrichtung zur Erstellung von Ceph-Aufnahmen in einer schaubildlichen Darstellung.

Am höhenverstellbaren Teil la der Tragsäule 1 (Fig. 2) ist ein Auslegerarm 6 befestigt, der eine (zweite) Kopfhalte- und Positoniereinrichtung 7 trägt. Der Auslegerarm 6 umfaßt ein Gehäuse 8, an dem ein Schwert 9, welches die Kopf-halte- und Positioniereinrichtung trägt, mit Hilfe von im Gehäuse 8 angeordneten Führungsrollen 10 verstellbar gelagert ist. Die Zeilenkamera 4 ist mittels eines Querträgers 11 mit einer Vorblende 12 verbunden, die dazu dient, den an sich schon in bekannter Weise von der der Röntgenstrahlenquelle 3 benachbarten Sekundärblende begrenzten Fächerstrahl nochmals exakt auf die Schlitzbreite und -länge der nachfolgend noch näher erläuterten Zeilenkamera zu justieren.

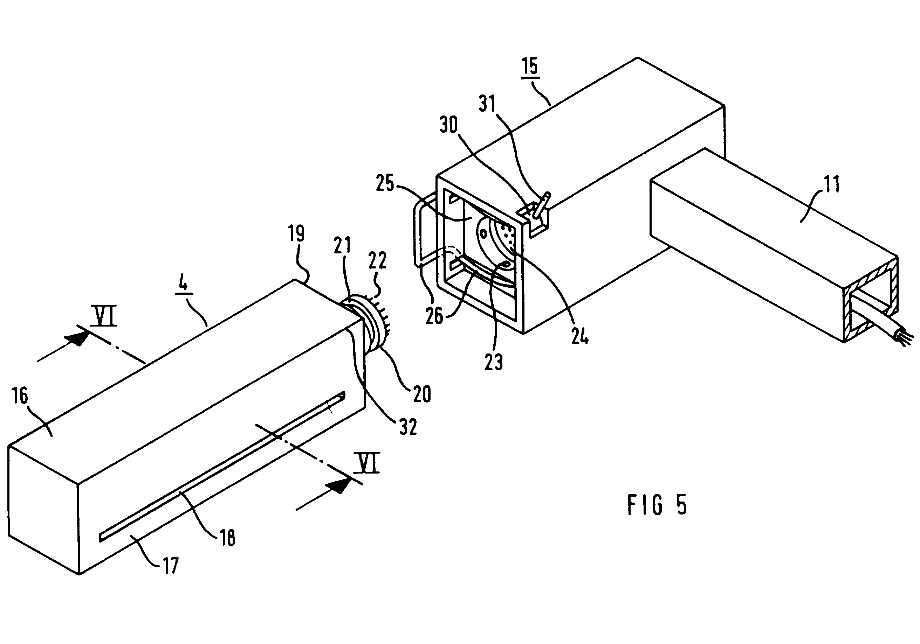

Im Gegensatz zum Ausführungsbeispiel nach Figur 1 ist die Zeilenkamera bei der Ausführung nach Figur 2 nicht vertikal, sondern waagerecht angeordnet. Eine entsprechend ausgebildete Halterung ist in Figur 5 dargestellt.

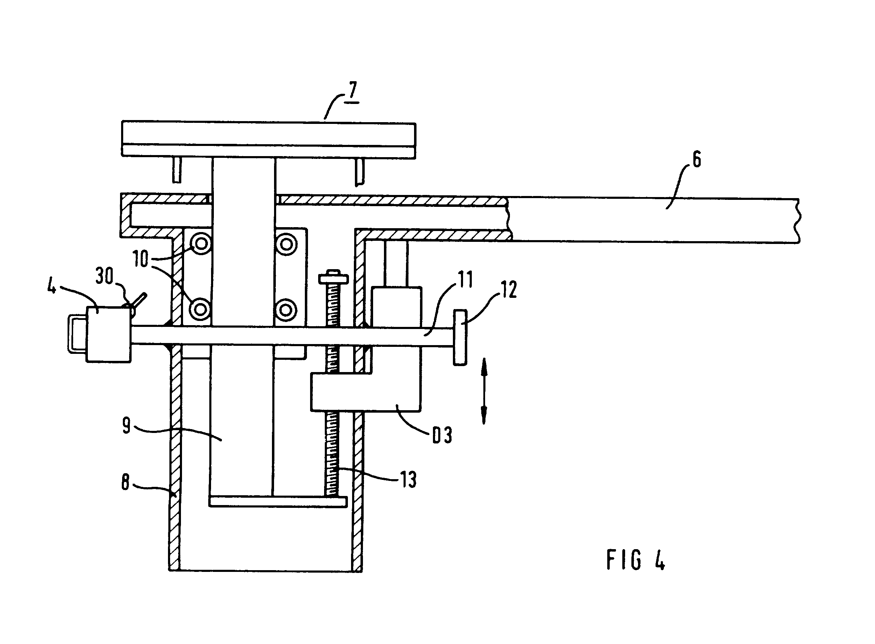

Wie aus Figur 4, die die Vorrichtung in Frontansicht und teilweise im Schnitt zeigt, ersichtlich, befindet sich am Schwert 9, welches die Kopfhalte- und Positioniereinrichtung trägt, eine Gewindespindel 13, die mit einem allgemein mit D3 bezeichneten Getriebemotor zusammenwirkt. Der Getriebemotor D3 ist entweder am Gehäuse 8 oder am Tragarm 6 befestigt. Wie später noch näher erläutert, wird mit Hilfe der aufgezeigten Verstellanordnung mit dem Antrieb D3 erreicht, daß, wenn die Röntgenstrahlenquelle 3 zusammen mit der Zeilenkamera 4 in der Vertikalen bewegt wird, die Kopfhalte- und Positioniereinrichtung 7 während der Ceph-Aufnahme effektiv keine Bewegung ausführt, d.h. ortsfest im Raum gehalten wird.

Die Figur 5 zeigt in einer schaubildlichen Explosionsdarstellung einerseits die Zeilenkamera 4 und andererseits eine mit 15 bezeichnete Halterung, die im vorliegenden Anwendungsfalle für die Ausführung nach Figur 2 am Querträger 11 befestigt ist. Im Falle der Version nach Figur 1 (für PAN-Aufnahmen) ist eine gleich ausgebildete Halterung (ohne Querträger 11) senkrecht an der Dreheinheit 2 (Figur 1) befestigt.

Die Zeilenkamera 4 enthält ein längliches Gehäuse 16, welches im Ausführungsbeispiel aus einem Vierkantrohr besteht und in der vorderen, der Strahlenquelle 3 zugewandten Seitenfläche 17 einen Schlitz 18 aufweist. Der Schlitz 18 befindet sich im unteren Drittel der Seitenfläche 17, wodurch die Zeilenkamera in eine vergleichsweise tiefe Ausgangsposition (sh. gestrichelte Darstellung in Fig. 2) gefahren werden kann.

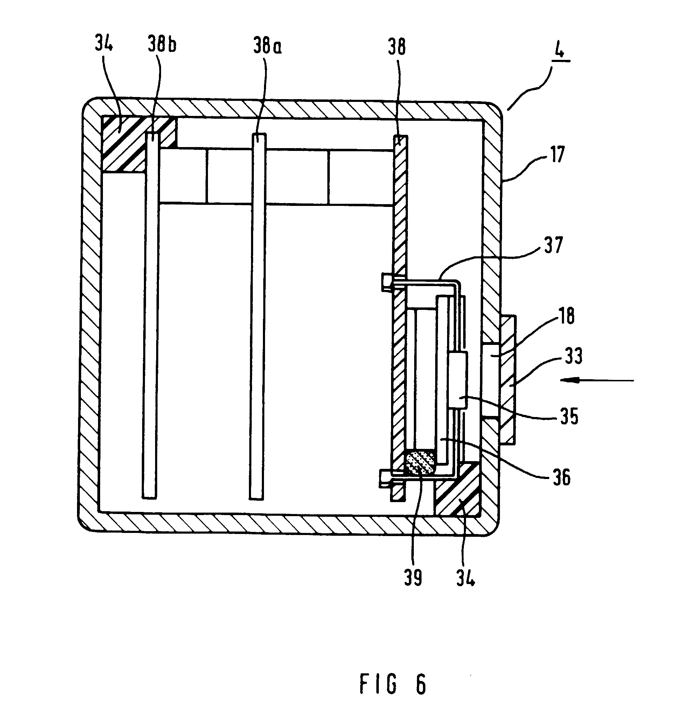

Wie aus Figur 6 noch näher hervorgeht, befindet sich hinter dem Schlitz 18 im Innern des Profilrohres 16 ein strahlenempfindlicher Zeilendetektor, z.B. in Form eines CCD-Sensors. An der einen Stirnseite 19 befindet sich ein zapfenförmiges Anschlußelement 20, welches mechanische und elektrische Anschlußmittel für eine elektrische und mechanische Verbindung mit dem Halter 15 aufweist. Die mechanischen Anschlußmittel enthalten eine Ringnut 21, die mit einer Kugelrastung 23 zusammenwirkt. Die elektrischen Anschlußmittel bestehen aus einem Mehrstiftstecker 22, der mit einer Steckbuchse 24 im Halter 15 zusammenwirkt. Die Steckstifte 22 sind mit dem bereits erwähnten Zeilendetektor und einer weiteren, im Inneren der Zeilenkamera 4 befindlichen Elektronik verbunden. Der Halter 15 ist so aufgebaut, daß bei aufgesetztem Zeilendetektor die Stirnseite 19 der Zeilenkamera 4 einer stirnseitigen Anschlußfläche 25 der Halterung 15 gegenübersteht.

Damit das Lösen der Zeilenkamera 4 von der Halterung 15 erleichtert ist, insbesondere ein Verkanten und damit die Gefahr einer Beschädigung der hochempfindlichen elektrischen Kontakte vermieden wird, ist eine Auswurfeinrichtung 26 vorgesehen. Diese besteht in der vorliegenden Ausführungsform aus einem Bügel, der in einem Schlitz der Gehäusewandung des Halters 15 nach außen geführt ist. Wird der Bügel bei aufgesetzter Zeilenkamera betätigt, drücken anliegende Bügelteile gegen die Stirnfläche 19 und üben so eine zentrische Kraft auf die Fläche aus, wodurch die Verbindung leicht gelöst werden kann.

Mit 30 ist eine Zentriereinrichtung bezeichnet, die einen exzentrisch im Gehäuse der Halterung gelagerten Hebel 31 enthält. Nach Einsetzen der Zeilenkamera 4 in den Halter 15 wird der Exzenterhebel 31 betätigt, wodurch eine Fläche des Exzenters auf die in der Figur mit 32 bezeichnete Kante des Gehäuses drückt und dieses in definierter, reproduzierbarer Position hält. Obgleich im vorliegenden Ausführungsbeispiel das Gehäuses einteilig ausgebildet ist, kann das Gehäuse auch mehrteilig ausgeführt sein, wobei der eine, den Detektor tragende Gehäuseteil dann in der vorgenannten Weise zentriert wird. Damit kann der Detektor unabhängig vom Kameragehäuse und dessen etwaigen Montage- und Fertigungstoleranzen in bezug auf den Halter fixiert werden.

Aus Figur 6, die einen Schnitt entlang der Linie VI-VI in Figur 5 zeigt, geht der prinzipielle Aufbau der Zeilenkamera hervor. Das Gehäuse ist lichtdicht ausgebildet; der Schlitz 18 ist stirnseitig von einer lichtdichten, aber röntgenstrahlendurchlässigen Kunststoffplatte 33 bedeckt. Dahinter befindet sich im Innern ein mit einer vorgeschalteten Szintillationsschicht und gegebenenfalls mit einer zwischengeschalteten Faseroptik versehener CCD-Sensor 35. Der CCD-Sensor 35 kann ein- oder mehrteilig und vorteilhafterweise als Sensormatrix aus amorphem Silizium ausgebildet sein. Ein metallischer Halter 37 verbindet den Träger 36 und das CCD-Element 35 mit einer Platine 38. Flexible Kontaktstreifen 39, z.B. aus mit Goldfasern versehenem Silicon, bewirken den elektrischen Kontakt zwischen Sensor 35 und Platine 38. Die Platine 38 enthält sämtliche, zur Ansteuerung des CCD-Sensors unmittelbar erforderlichen Bauelemente. Gegebenenfalls sind im Gehäuse noch weitere Platinen 38a, 38b angeordnet. Die von der (den) Platine(n) 38 (38a, 38b) abgehenden Leitungen führen zu den bereits erwähnten Steckstiften 22 (Fig. 5). Mit 34 sind stoßabsorbierende Elemente bezeichnet, die den Detektor 35 und die Steuerplatinen 38, 38a, 38b im Gehäuse 'schwimmend' lagern. Damit lassen sich die hochempfindlichen und teueren Teile bei einem unbeabsichtigten Herabfallen der Kamera vor Bruch bzw. Lösen der Kontaktverbindungen weitgehend schützen.

Wie eingangs bereits erwähnt, ist für PAN-Aufnahmen (Fig. 1) und Ceph-Aufnahmen (Fig. 2) das gleiche Grundgerät und ein und dieselbe Kamera verwendbar. Um die für eine Ceph-Aufnahme nötige Bildgröße zu erreichen, hat die Zeilenkamera vorteilhafterweise einen entsprechend längeren Sensor. Die Zeilenkamera kann so je nach Bedarf entweder am Ceph- oder am PAN-Halter angebracht werden. Zur Halterung der Zeilenkamera am Halter 15 sind verschiedene Möglichkeiten denkbar. Anstelle der gezeigten Kugelrastung kann auch eine Bajonettverbindung vorgesehen sein. Desgleichen kann anstelle eines Vierkantprofiles eine andere äußere Formgestaltung für das Gehäuse der Zeilenkamera vorgesehen sein.

Zum Aufnahmeprinzip wird folgendes angemerkt:
Eine PAN-Schichtaufnahme wird in der Weise erzielt, daß die beim Überstreichen des aufzunehmenden Objekts (Kiefer) gewonnenen Signale in dem zweidimensional auflösenden Detektor aufaddiert werden, wobei das Aufaddieren der Signale - falls ein CCD-Sensor verwendet wird - bereits auf dem Sensor durchgeführt werden kann, indem dieser im TDI-Modus betrieben wird. Durch diese besondere Betriebsart wird die Funktion eines bewegten Filmes nachgebildet, indem die durch Belichtung erzeugten Ladungspakete im CCD-Element entsprechend weitergetaktet werden, während ständig neue Ladungen hinzukommen. Die Taktimpulse für den TDI-Betrieb werden aus den sonst für den Filmkassettenantrieb erforderlichen Schrittmotorimpulsen abgeleitet.

Alternativ ist auch ein Aufaddieren in einer späteren Signalverarbeitungsstufe möglich.

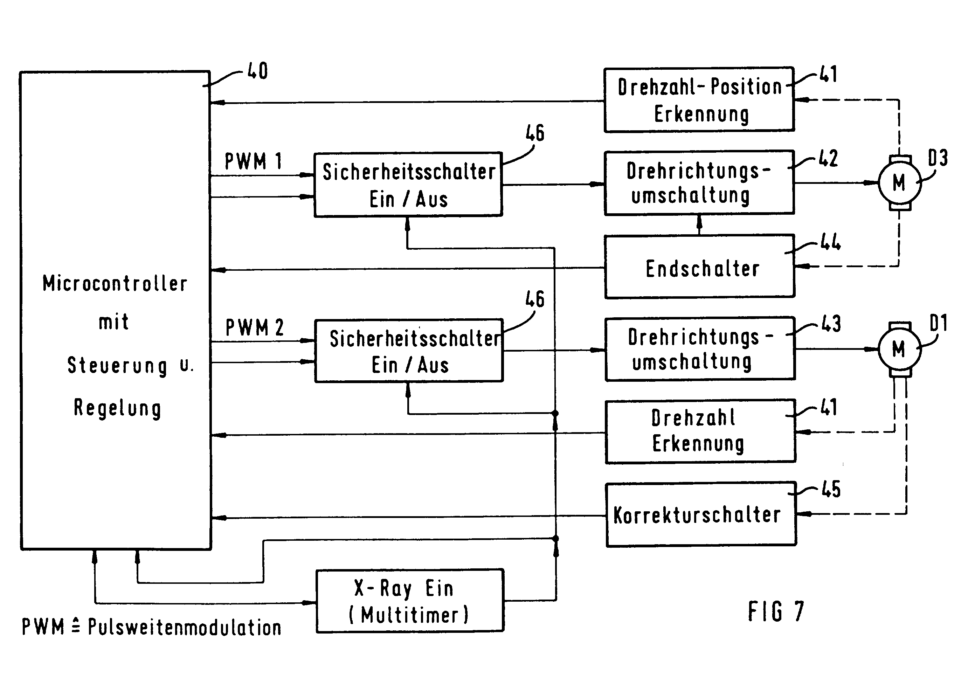

Die Ceph-Aufnahme läuft ebenfalls in Slot-Technik ab. Der Kopf eines stehenden (oder sitzenden) Patienten wird je nach Anordnung der Zeilenkamera von oben nach unten (bei waagerechter Anordnung) oder von links nach rechts (bei senkrechter Anordnung) vice versa mit einem Strahlfächer überstrichen. Dieser Strahlfächer trifft, justiert durch die bereits genannte Vorblende 11, genau auf den waagerecht angeordneten Schlitz des CCD-Sensors. Mit Hilfe des Antriebs D1 verfährt man nun das gesamte Gerät, also Röntgenstrahlenquelle 3 mit Primär- und Sekundärblende sowie Zeilenkamera 4 mit Sensor, gemeinsam von einer Ausgangsposition aus in der Vertikalen (sh. Pfeile in Fig. 2). Gleichzeitig wird die Kopfhalte- und Positioniereinrichtung 7 mit Hilfe des Antriebs D3 in Gegenrichtung gefahren, wobei die beiden Bewegungen so aufeinander abgestimmt sind, daß der Patientenkopf räumlich fixiert, d.h. ortsfest, bleibt. Die Steuerung der beiden Antriebsmotoren D1 und D3 erfolgt entsprechend dem Blockschaltbild nach Figur 7 über einen Mikroprozessor 40. Den beiden Antrieben sind Drehzahlerkennungssensoren 41, Drehrichtungsumschalter 42 sowie End-bzw. Korrekturschalter 44, 45 zugeordnet. Die über Pulsweitenmodulation erfolgte Steuerung enthält weiterhin Sicherheitsschalter 46. Eine Auswerteelektronik des Mikro-Controllers 40 erkennt, an welcher Halterung (PAN- oder Ceph-Gerät) die Kamera befestigt ist. Wird eine Ceph-Aufnahme angewählt, fährt der Antriebsmotor D3 in die Ausgangsposition, beispielsweise in die untere Verstellposition (gestrichelte Position in Fig. 2). In dieser Position spricht der Endschalter 44 an. Mittels der Höhenverstellung der Tragsäule 1 kann nun die Kopfhaltepositoniereinrichtung auf die Patientengröße eingestellt werden. Während der Ceph-Aufnahme verfährt der Antriebsmotor D3 die Kopfpositioniereinrichtung 7 nach oben, während gleichzeitig der Antriebsmotor D1 für die Tragsäule nach unten fährt. Die beiden Antriebe werden dabei so geregelt, daß die Differenz der Verstellgeschwindigkeiten gleich Null ist. Dadurch ist sichergestellt, daß der Abstand der Ohroliven und damit der Kopfpositionierung zum Fußboden konstant bleibt. Die Aufnahme ist beendet, wenn der Endschalter 44 oder ein Systemtakt-Zähler (TDI-Takt-Zähler) die obere Endlage erkennt.

Der TDI-Takt für den CCD-Sensor wird z.B. vom Antriebsmotor D1, der für die Höhenverstellung der Tragsäule vorgesehen ist, abgeleitet. Alternativ kann er auch aus den Signalen eines Positionszählers, der die Verstellung der Tragsäule direkt mißt, gewonnen werden. Der TDI-Modus dient hier nicht, wie bei einer PAN-Aufnahme, um eine Verwischung und damit eine Schichtaufnahme zu erzeugen, sondern dazu, die volle Breite des Sensors zur Bildentstehung auszunutzen. Hier entspricht also der TDI-Betrieb einem Film, der relativ zum Schlitz bewegt wird und relativ zum Patienten feststeht.

## Patentansprüche

1. Zeilendetektor-Kamera (4), die dazu vorgesehen ist, an einem diametral zur Strahlenquelle (3) eines Röntgendiagnostikgerätes (2) zur Erstellung von Objektaufnahmen eines Patienten angeordneten Halter (15) angeschlossen zu werden und die folgende Merkmale aufweist:
- die Kamera enthält ein langgestrecktes Gehäuse (16), welches an einer der Strahlenquelle (3) zugewandten Fläche (17) einen in Längsrichtung sich erstreckenden Schlitz (18) für den Eintritt der Röntgenstrahlung aufweist;
- hinter dem Schlitz (18) ist ein röntgenstrahlenempfindlicher Detektor (35) angeordnet, der eine Vielzahl von Detektorelementen aufweist, die so angeordnet sind, daß sie eine oder mehrere Zeilen bilden;
- die Kontakte der Detektorelemente sind mit Steuerleitungen verbunden, welche zu einem am einen Ende des Gehäuses (16) angeordneteten Anschlußteil (20) geführt sind;
- das Anschlußteil (20) enthält elektrische und mechanische Anschlußmittel (21, 23, 22, 24) für den lösbaren Anschluß an den entsprechend ausgebildeten Halter (15) des Röntgendiagnostikgerätes (Fig. 1, Fig. 2).

2. Zeilendetektor-Kamera nach Anspruch 1, bei der das Anschlußteil (20) mit einer Verriegelungsmechanik (21, 23) gegen unbeabsichtigtes Lösen vom Halter (15) versehen ist.

3. Zeilendetektor-Kamera nach Anspruch 1, bei der das Kameragehäuse (16) und/oder der Halter (15) ein Zentrierungsmittel (30, 31) beinhalten, mit dem das Kameragehäuse (16) oder Teile davon in bezug auf den Halter (15) in einer definierten Position fixierbar ist/sind.

4. Zeilendetektor-Kamera nach Anspruch 3, bei der das Zentrierungsmittel durch Gestaltung der äußeren Form des Kameragehäuses (16) gegeben ist, derart, daß eine unverwechselbare Zuordnung des Anschlußteils (20) an den Halter (15) des Röntgendiagnostikgerätes gegeben ist.

5. Zeilendetektor-Kamera nach einem der Ansprüche 1 bis 4, bei der die äußere Form des Kameragehäuses (16) durch ein Mehrkantprofilrohr gegeben ist, an dessen einem stirnseitigen Ende sich das Anschlußteil (20) befindet.

6. Zeilendetektor-Kamera nach einem der Ansprüche 1 bis 5, bei der die das Anschlußteil (20) enthaltende stirnseitige Fläche (19) mit einer am Halter (15) angeordneten Hilfseinrichtung (26) zusammenwirkt, die so gestaltet ist, daß bei deren Betätigung die Anschlußmittel axial voneinander gelöst werden.

7. Zeilendetektor-Kamera nach einem der Ansprüche 1 bis 6, die ein Hohlprofilgehäuse (16) von definierter, etwa der Schädelbreite eines Patienten entsprechenden Länge aufweist, welches an der einen, der Strahlenquelle zugewandten Längsseite (17) einen längs verlaufenden Schlitz (18) aufweist, hinter dem im Innern des Gehäuses als Detektor (35) ein CCD-Sensor mit Szintillatorschicht angeordnet ist und wobei im Hohlprofilgehäuse (16) mindestens eine mit elektrischen Bauteilen besetzte Steuerplatine (38) zur Ansteuerung des CCD-Sensors enthalten ist.

8. Zeilendetektor-Kamera nach Anspruch 7, bei der das Gehäuse (16) als ein Vierkant-Hohlprofilrohr (16) mit vorzugsweise quadratischen Querschnitt ausgebildet ist.

9. Zeilendetektor-Kamera nach Anspruch 7, bei der der CCD-Sensor an einem Träger (36) befestigt ist, der mittels Halteklammern (37) mit der einen Steuerplatine (38) verspannt ist.

10. Zeilendetektor-Kamera nach einem der Ansprüche 1 bis 9, enthaltend eine mit dem Halter (15) zu verbindende Auffangsicherung gegen unbeabsichtigtes Herabfallen nach Lösen der Anschlußmittel (21-24).

11. Zeilendetektor-Kamera nach einem der Ansprüche 7 bis 10, bei der der Detektor (35) und/oder die Steuerplatine (38) mittels eines stoßabsorbierenden Dämpfungselements (34) im Gehäuse (16) gehaltert sind/ist.

12. Zeilendetektor-Kamera nach einem der bisherigen Ansprüche, in Verbindung mit einem PAN- und/oder Ceph-Gerät (Fig. 1/2).

## Claims

1. A line detector camera (4) intended for being connected to a holder (15) which is arranged diametrically opposite to the x-ray source (3) of an x-ray diagnostics installation (2) for generating object exposures of a patient, having the following features:
- the camera has an elongated housing (16) which, in a surface (17) facing the x-ray source, has a longitudinally extending slot (18) therein for the entry of x-rays;
- an x-ray sensitive detector (35) is disposed behind the slot (18), said detector (35) having a plurality of detector elements arranged to form one or several line(s);
- the contacts of the detector elements are connected to control lines which are carried to a connector part (20) disposed at an end of the housing (16);
- the connector part (20) contains electrical and mechanical connector means (21, 23, 22, 24) for a releasable connection to the correspondingly designed holder (15) of the x-ray diagnostics installation (Fig. 1, Fig. 2).

2. A line detector camera according to claim 1, wherein the connector part (20) further includes an interlock mechanism (21, 23) for preventing unintentional release from the holder (15).

3. A line detector camera according to claim 1, wherein the housing (16) and/or the holder (15) contain centering means (30, 31) for fixing the housing (16) or parts thereof in a defined position relative to the holder (15).

4. A line detector camera according to claim 3, wherein the centering means is given by forming the outer shape of the camera housing (16) in such a way that there is an unmistakable relation between the connector part (20) and the holder (15) of the x-ray diagnostics installation.

5. A line detector camera according to one of claims 1 to 4, wherein the outer shape of the housing (16) is that of a multi-cornered hollow tube at whose one end face the connector part (20) is disposed.

6. A line detector camera according to one of claims 1 to 5, wherein the end face (19) containing the connector part (20) acts in combination with an auxiliary device (26) attached to the holder (15) which is designed in such a way that the connector means are axially released from each other upon actuation of the auxiliary device (26).

7. A line detector camera according to one of claims 1 to 6, which has a hollow-tube housing (16) of a defined length approximately corresponding to the width of a human skull, wherein the housing (16) has a longitudinally extending slot (18) in a side wall (17) facing the x-rays, behind which a CCD sensor with a scintillator layer is disposed as a detector (35) inside the housing, and wherein at least one control circuit board (38) for driving the CCD sensor, containing electrical components, is contained in the hollow-tube housing (16).

8. A line detector camera according to claim 7, wherein the housing (16) is shaped as a rectangular hollow tube preferably having a square cross section.

9. A line detector camera according to claim 7, wherein the CCD sensor is attached to a carrier (36) which is braced to the one control circuit board (38) with retainer clamps (37).

10. A line detector camera according to one of claims 1 to 9, further comprising a safety catch means to be attached to the holder (15) for preventing unintentional dropping after release of the connector means (21-24).

11. A line detector camera according to one of claims 7 to 10, wherein the detector (35) and/or the control circuit board (38) are/is mounted in the housing (16) by means of a shock-absorbing damping means (34).

12. A line detector camera according to one of the preceding claims, in combination with a PAN and/or Ceph device (Fig. 1/2).

## Revendications

1. Caméra à balayage de ligne (4), qui est prévue pour être raccordée à une console (15), disposée de manière diamétralement opposée à la source de rayonnement (3) d'un appareil de diagnostic aux rayons X (2) pour la prise de vues d'un patient, et qui présente les particularités suivantes :
- la caméra comprend un boîtier (16) allongé, qui présente, sur une surface (17) dirigée vers la source de rayonnement (3), une fente (18) qui s'étend dans le sens de la longueur pour l'entrée des rayons X ;
- un détecteur (35), sensible aux rayons X, est placé derrière la fente (18) et il comprend une pluralité d'éléments détecteurs, qui sont disposés de manière à former une ou plusieurs ligne(s) ;
- les contacts des éléments détecteurs sont reliés à des lignes de commande, qui sont menées jusqu'à un élément de raccordement (20) placé au niveau d'une extrémité du boîtier (16) ;
- l'élément de raccordement (20) contient des moyens de raccordements électriques et mécaniques (21, 23, 22, 24) pour une fixation, de manière détachable, à la console (15), réalisée de forme correspondante, de l'appareil de diagnostic aux rayons X (figure 1, figure 2).

2. Caméra à balayage de ligne selon la revendication 1, dans laquelle l'élément de raccordement (20) est pourvu d'un mécanisme de verrouillage (21, 23) contre tout débranchement involontaire par rapport à la console (15).

3. Caméra à balayage de ligne selon la revendication 1, dans laquelle le boîtier de caméra (16) et/ou la console (15) comporte(nt) un moyen de positionnement (30, 31) permettant d'immobiliser, dans une position définie, le boîtier de caméra (16) ou des parties de celui-ci par rapport à la console (15).

4. Caméra à balayage de ligne selon la revendication 3, dans laquelle le moyen de positionnement est obtenu par façonnage de la forme extérieure du boîtier de caméra (16), de telle manière que soit obtenue une coordination irréversible de l'élément de raccordement (20) et de la console (15) de l'appareil de diagnostic aux rayons X.

5. Caméra à balayage de ligne selon l'une des revendications 1 à 4, dans laquelle la forme extérieure du boîtier de caméra (16) est définie par un tube profilé polygonal, à l'extrémité frontale duquel se trouve l'élément de raccordement (20).

6. Caméra à balayage de ligne selon l'une des revendications 1 à 5, dans laquelle la surface frontale (19), contenant l'élément de raccordement (20), coopère avec un dispositif auxiliaire (26), situé sur la console (15), et réalisé de telle manière que, lors de son actionnement, les moyens de raccordement soient axialement détachés l'un de l'autre.

7. Caméra à balayage de ligne selon l'une des revendications 1 à 6, qui présente un carter profilé creux (16) de longueur définie, correspondant à peu près à la largeur du crâne d'un patient, et qui présente, sur un grand côté (17), dirigé vers la source de rayonnement, une fente (18) qui s'étend dans le sens longitudinal et derrière laquelle, est disposé, à l'intérieur du carter, un capteur à CCD (à couplage de charges) à couche à scintillation, en tant que détecteur (35), et en ce qu'il est prévu, dans le carter profilé creux (16), au moins une plaquette de commande (38) garnie de composants électriques, pour l'attaque du capteur à CCD.

8. Caméra à balayage de ligne selon la revendication 7, dans laquelle le carter (16) est réalisé en tant que tube profilé creux à quatre pans (16) avec, de préférence, une section transversale carrée.

9. Caméra à balayage de ligne selon la revendication 7, dans laquelle le capteur à CCD est fixé sur un support (36), qui est bloqué sur la plaquette de commande (38) au moyen d'étriers de retenue (37).

10. Caméra à balayage de ligne selon l'une des revendications 1 à 9, comprenant un dispositif d'arrêt de sécurité, à relier à la console (15), afin de prévenir une chute inopinée après séparation des moyens de raccordement (21 à 24).

11. Caméra à balayage de ligne selon l'une des revendications 7 à 10, dans laquelle le détecteur (35) et/ou la plaquette de commande (38) est ou sont maintenu(s) dans le carter (16) à l'aide d'un élément d'amortissement (34) absorbant les chocs.

12. Caméra à balayage de ligne selon l'une des revendications précédentes, en association avec un appareil à prise de vues panoramiques et/ou du crâne (figures 1/2).
